# FASCICULE DE BREVET EUROPEEN

(11) **EP 0 912 591 B1**
(45) Date de publication et mention de la délivrance du brevet: **13.02.2002**
(21) Numéro de dépôt: 97924078.5
(22) Date de dépôt: 13.05.1997
(51) Int. Cl.: C07H 17/08, A61K 31/70

(54) **NOUVEAU PROCEDE D'ISOMERISATION DU RADICAL METHYLE EN 10 DE DERIVES DE L'ERYTHROMYCINE**
VERFAHREN ZUR ISOMERISIERUNG VON METHYL-TEIL IN DER 10-POSITION VON ERYTHROMYCIN
NOVEL METHOD FOR ISOMERISING THE 10-METHYL RADICAL OF ERYTHROMYCIN DERIVATIVES

(30) Priorité: 14.05.1996 FR 9605966
(43) Date de publication de la demande: 06.05.1999
(73) Titulaire: Aventis Pharma S.A., 92160 Antony (FR)
(72) Inventeur: BENEDETTI, Yannick, F-93110 Rosny sous Bois (FR); LAGOUARDAT, Jacques, F-93160 Noisy le Grand (FR); SCHOLL, Jacques, F-93230 Romainville (FR)
(86) Numéro de dépôt international: FR9700840
(87) Numéro de publication internationale: WO9743297

(56) Documents cités:
- EP-A- 0 676 409

## Description

L'invention concerne un nouveau procédé d'isomérisation du radical méthyle en 10 des dérivés de l'érythromycine.

L'invention a pour objet un procédé d'isomérisation caractérisé en ce que l'on soumet un composé de formule (I_{A}) : 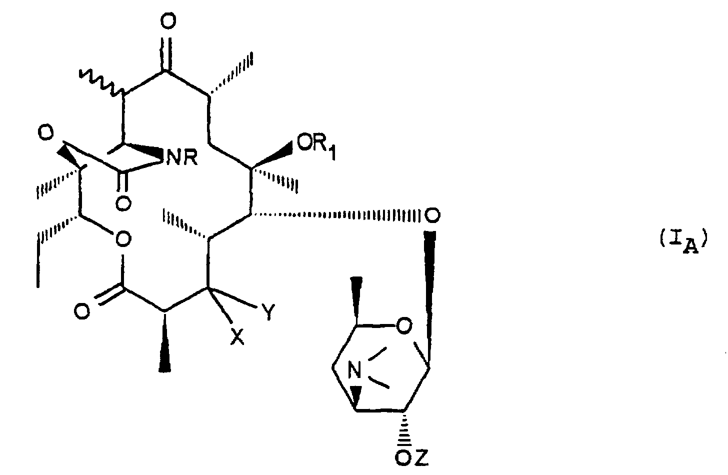 dans laquelle ou bien X et Y forment ensemble un radical 3-oxo, ou bien X représente un atome d'hydrogène et Y représente soit un radical : 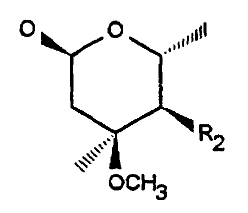 dans lequel R₂ représente un radical hydroxyle ou un radical 0-acyle renfermant de 2 à 20 atomes de carbone, soit un radical O-alkyle, renfermant de 2 à 20 atomes de carbone, soit un radical NH₂,
- R₁ représente un atome d'hydrogène ou un radical méthyle,
- Z représente un hydrogène ou un radical acyle renfermant de 2 à 20 atomes de carbone,
- R représente un atome d'hydrogène, un radical NH₂ ou un radical (CH₂)ₙAr, NH(CH₂)ₙAr ou N=CH(CH₂)ₙAr dans lequel n représente un nombre entier compris entre 1 et 6, et Ar représente un radical aryle ou hétéroaryle éventuellement substitué, sous forme d'isomère 10α ou de mélange d'isomères 100α et 10β, à l'action d'un agent basique pour obtenir le composé de formule (I) correspondant dans lequel le radical méthyle en 10 est en position β : 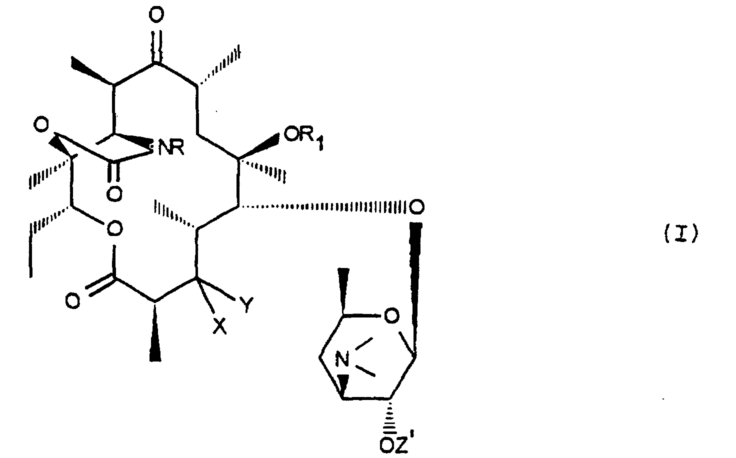 et dans laquelle R, R₁, X et Y conservent leur signification précédente et Z' représente un atome d'hydrogène ou un radical acyle renfermant de 2 à 20 atomes de carbone.

Le radical acyle est de préférence un radical acétyle, propionyle, butyryle, isobutyryle, n-valéryle, isovaléryle, tervaléryle et pivalyle, ou un radical benzyle.

Par radical aryle, on entend de préférence un radical phényle ou naphtyle, par radical hétéroaryle, on entend un radical renfermant un ou plusieurs hétéroaromes choisis de préférence parmi l'oxygène, le soufre ou l'azote, il peut s'agir d'un radical thiényle, furyle, pyrrolyle, thiazolyle, oxazolyle, imidazolyle, thiadiazolyle, pyrazolyle, ou isoxazolyle, d'un radical pyridyle, pyridazinyle, ou pyrazinyle, d'un radical indolyle, benzofuryle, benzothiényle, guinoléinyle ou pyridyl-imidazolyle.

Comme radical hétéroaryle, on peut citer par exemple les radicaux :

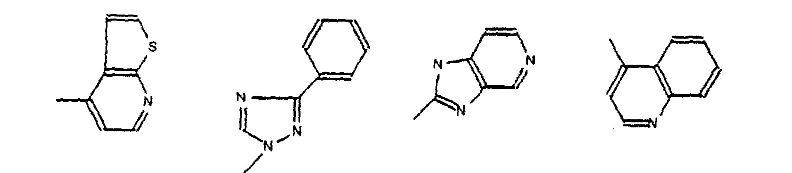

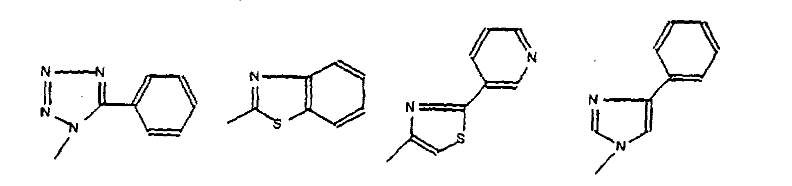

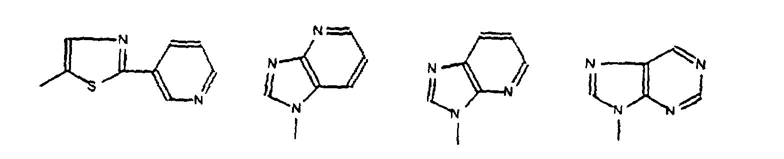

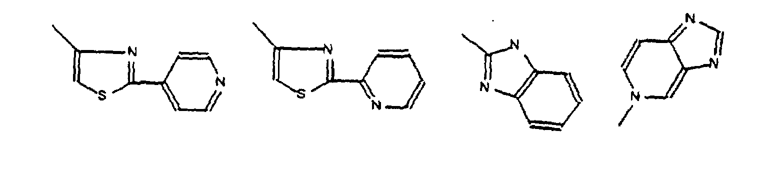

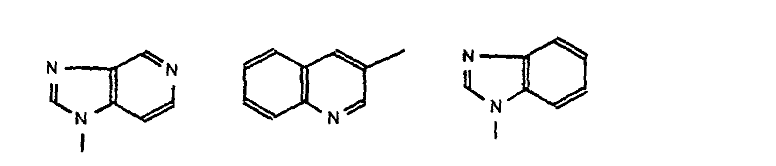

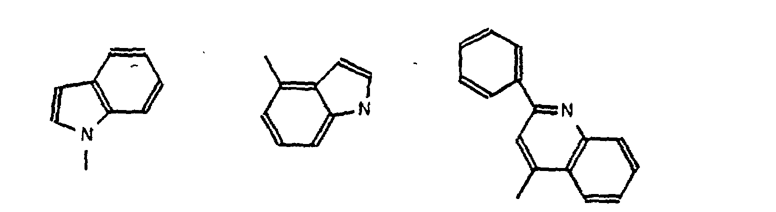

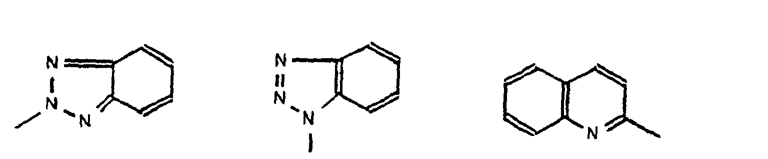

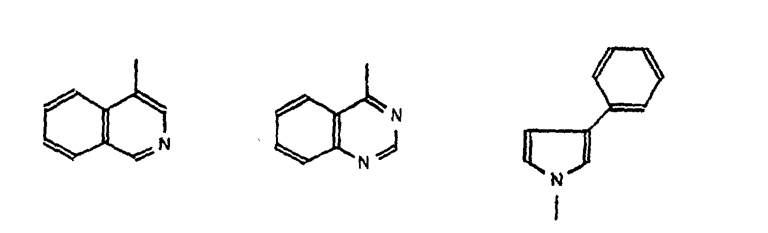

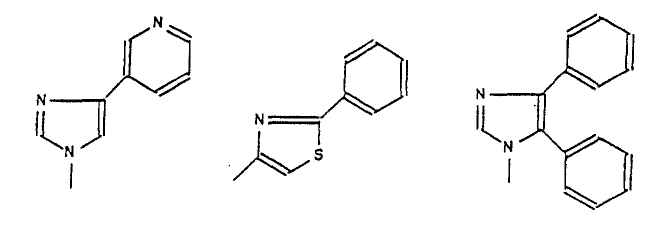

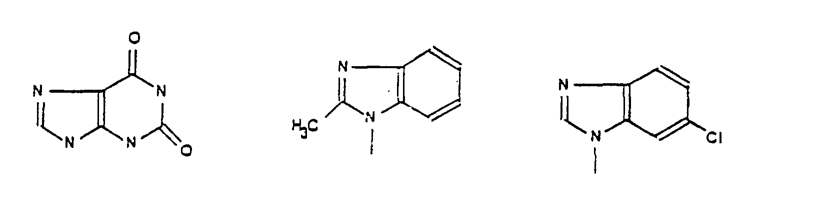

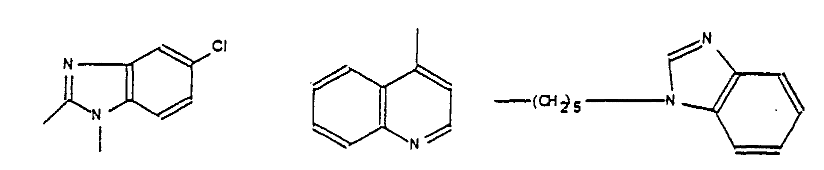

L'invention a plus particulièrement pour objet un procédé caractérisé en ce que l'on opère en présence d'un agent basique, de préférence en quantité catalytique, Z et Z' représentant un atome d'hydrogène.

L'agent basique est de préférence la potasse ou encore un hydroxyde de tétra-alkylammonium par exemple l'hydroxyde ou le bromure de tétrabutylammonium ou le DBU (1,8-diazabicyclo [5-4-0]undec-7-ène) ou un carbonate alcalin par exemple le carbonate de sodium ou de potassium ou la soude ou le phosphate tripotassique ou encore le méthylate de sodium.

Il peut être nécessaire dans certains cas d'ajouter un agent de transfert de phase, par exemple le bromure de. tétrabutyl ammonium.

L'invention a tout spécialement pour objet un procédé caractérisé en ce que l'on opère au sein d'un solvant qui peut être par exemple le tétrahydrofuranne, la 1-méthyl 2-pyrrolidonone en solution aqueuse, le chlorure de méthylène et plus spécialement un alcool en particulier le méthanol et obtient ainsi le composé correspondant dans lequel Z' représente un atome d'hydrogène, ou un radical acyle renfermant de 2 à 20 atomes de carbone.

L'invention a plus particulièrement pour objet :
- un procédé d'isomérisation de composés de formule (I_{A}) en (I) dans lequel R représente un radical NH₂,
- un procédé d'isomérisation de composés de formule (I_{A}) en (I) dans lequel R₁ représente un radical méthyle,
- un procédé d'isomérisation de composés de formule (I_{A}) en (I) caractérisé en ce que X et Y forment ensemble un radical 3-oxo,
- un procédé d'isomérisation de composés de formule (I_{A}) en (I) dans lequel Z et Z' représentent un atome d'hydrogène.

L'invention concerne donc un procédé qui permet de transformer des composés de formule (I_{A}) dans lesquels le radical méthyle en 10 est en position α ou un mélange de 10α et 10β en composés de formule (I) dans lesquels le radical méthyle en 10 est en 10β. Lors de la formation de la chaîne en 11,12 on obtient un mélange d'isomères 10α et 10β, c'est ainsi que si l'on suit le procédé décrit dans le brevet EP 676409, on obtient la réaction suivante : 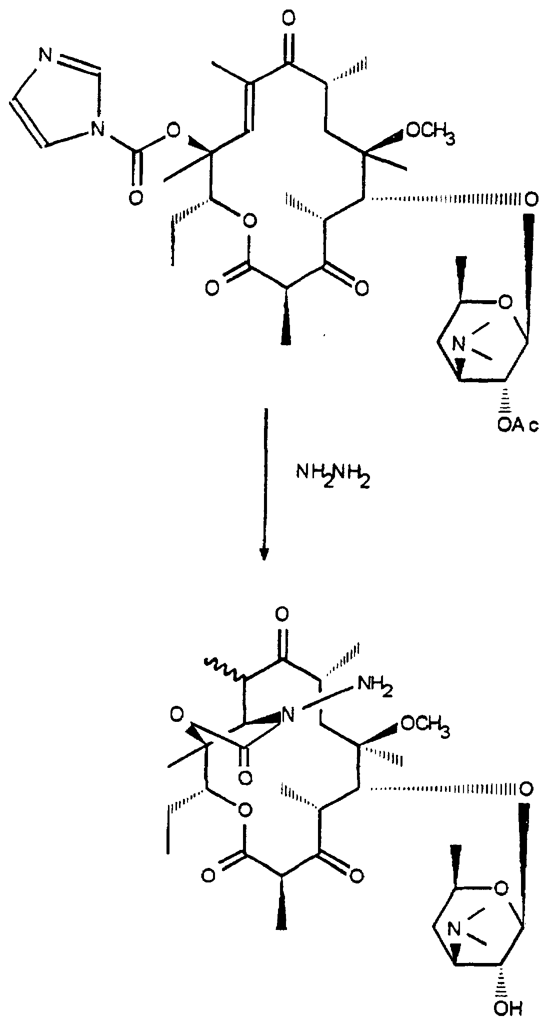 le produit (I_{A}) obtenu est un mélange d'isomère α et β, comme il est indiqué à l'exemple 1 du brevet EP 676409. Le produit 10β est un produit doué de propriétés antibiotiques intéressantes, il permet également la préparation d'autres produits antibiotiques décrits et revendiqués dans la demande de brevet 676409 selon le procédé :

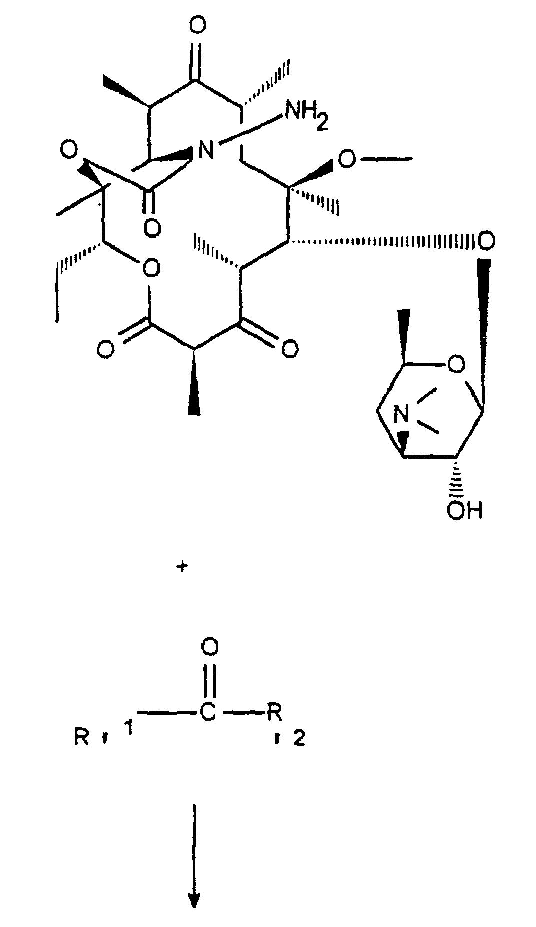

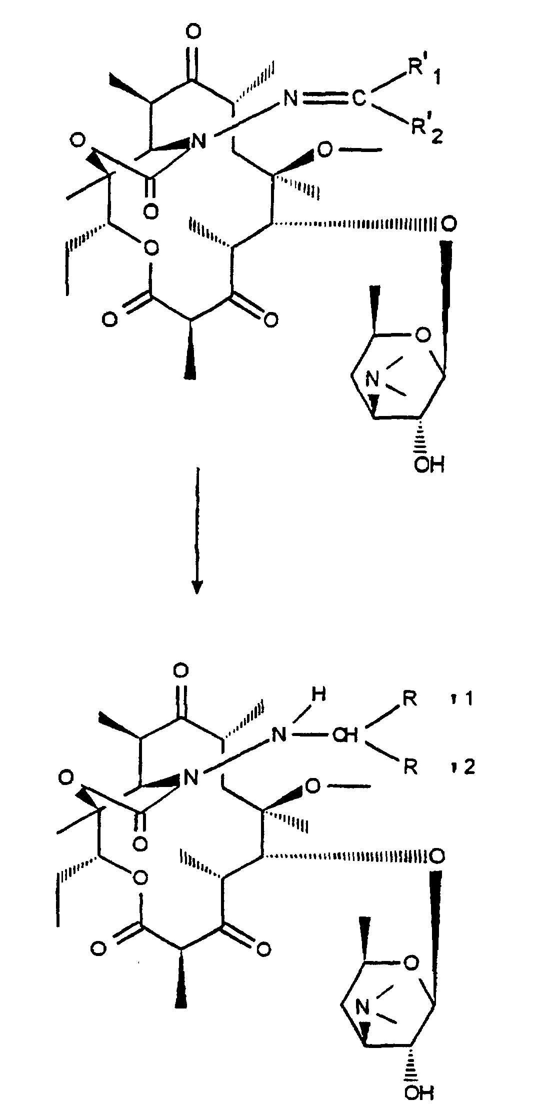

Les composés doués de propriétés antibiotiques intéressantes sont ceux dans lesquels le radical méthyle en 10 est en position β. Il est donc intéressant d'un point de vue industriel d'isomériser le produit 10α ou les mélanges 10α et 10β en produit 10β. Le procédé de l'invention permet notamment d'isomériser en isomère 10β les produits de formule (I_{A}) dans lesquels R est un radical NH₂, ceux dans lesquels R est un atome d'hydrogène, ou encore ceux dans lesquels R est un radical (CH₂)ₙAr, NH-(CH₂)ₙAr ou N=CH(CH₂)ₙAr dans lesquels n et Ar conservent leur signification précédente.

Ces produits sont décrits par exemple dans les demandes de brevet EP 676409, EP 638584, EP 680967, EP 0596802, EP 487411.

Les exemples suivants illustrent l'invention sans toutefois la limiter.

### EXEMPLE 1 :

On dissout dans 2 ml de méthanol 0,1 g de l'isomère 10α de la 11,12-didéoxy 3-de-((2,6-didéoxy 3-C-méthyl 3-O-méthyl alpha L-ribohexopyranosyl) oxy) 6-O-méthyl 3-oxo 12,11-(oxycarbonyl (hydrozono) érythromycine (préparé comme indiqué dans la demande de brevet européen 0676409, produit B isomères 10S) et ajoute 10 µl d'une solution de potasse méthanolique à 10 %. On agite une nuit à 20°C et observe la formation de l'isomère 10β. Cet isomère 10β est caractérisé par son spectre RMN.

Le rendement d'isomérisacion est de l'ordre de 90 %.

En opérant comme à l'exemple 1, on a obtenu l'isomère 10β du produit de l'exemple 1, à partir de 0,5 g du produit 10α :
- méthanol 10 volumes ; 0,11 ml de solution à 10 % de potasse dans le méthanol,
- méthanol 10 volumes ; 26 µl de solution à 40 % d'hydroxyde de tétrabutylammonium dans l'eau,
- méthanol 10 volumes ; 0,13 ml de solution à 40 % d'hydroxyde de tétrabutylammonium dans l'eau,
- méthanol à 20 % d'eau 10 volumes ; 0,11 ml de solution de KOH à 10 % dans le méthanol,
- méthanol à 20 % d'eau 10 volumes ; 26 µl de solution à 40 % d'hydroxyde de tétrabutylammonium dans l'eau,
- méthanol à 20 % d'eau 10 volumes ; 0,13 ml de solution à 40 % d'hydroxyde de tétrabutylammonium dans l'eau,
- méthanol à 20 % d'eau 10 volumes ; DBU 30 µl.

### EXEMPLE 2 : à partir du mélange d'isomères α + β

Dans un ballon tricol de 1000 ml muni d'une agitation, d'une sonde thermométrique et d'une arrivée d'azote en balayage, on introduit :
- mélange d'isomères α + β 37,8 g (isomère α = 15,8 g ; isomères β = 22 g)
   de la 11,12-didéoxy 3-de-((2,6-didéoxy 3-C-méthyl 3-O-méthyl α L-ribohexopyranosyl) oxy) 6-O-méthyl 3-oxo 12,11-(oxycarbonyl) hydrazono) érythromycine
   (isomère α = 15,8 g : isomère β = 22 g)
- méthanol pur anhydre
- potasse méthanolique à 10 %

On maintient sous agitation pendant 20 h à 20~22°C.

On vérifie par HPLC la disparition de l'isomère 10 α (< 2 %) puis introduit en contrôlant la température 20/22°C par un bain d'eau + glace :
- eau déminéralisée
   ajuste le pH à 10 avec acide acétique qs (-0,250 ml), agite 3 h à 20/22°C, refroidit à 0+2°C, agite 1 h à 0°+2°C, essore par clairçage à l'aide de :

- eau déminéralisée à 20°C
   Sèche en étuve ventilée à 30°C pendant 16 heures.
   On récupère l'isomère β pur : 32,6 g.
   Rendement : 86,2 % par rapport au mélane α + β.

### EXEMPLE 3 :

On dissout dans 1 ml de méthanol, 0,2 g d'un mélange des isomères 10α et 10β de la 11,12-didéoxy 3-de-((2,6-didéoxy 3-C-méthyl 3-O-méthyl α L-ribohexopyranosyl) oxy) 6-O-méthyl 3-oxo 12,11-(oxycarbonyl) hydrazono) érythromycine (%β/%α = 0,24) et ajoute 50 µl d'une solution de potasse méthanolique à 10%. On agite 16 heures à 20°C et observe la transformation de l'isomère 10α en isomère 10β.
(%β/%α = 21).

### EXEMPLE 4 :

On dissout dans 0,5 ml de méthanol, 0,1 g d'un mélange des isomères 10α et 10β de la 11,12-didéoxy 3-de-((2,6-didéoxy 3-C-méthyl 3-O-méthyl α L-ribohexopyranosyl) oxy) 6-O-méthyl 3-oxo 12,11-(oxycarbonyl) hydrazono) érythromycine (%β/%α = 0,24) et ajoute 0,25 mole par mole de (1,8- diazabicyclo [5-4-0] undec-7-ène. On agite 16 heures à 20°C et observe la transformation de l'isomère 100α en isomère 10β.
(%β/%α = 16,3).

### EXEMPLE 5 :

On dissout dans 0,5 ml de dichlorométhane, 0,1 g d'un mélange des isomères 10α et 10β de la 11,12-didéoxy 3-de-((2,6-didéoxy 3-C-méthyl 3-O-méthyl α L-ribohexopyranosyl) oxy) 6-O-méthyl 3-oxo 12,11-(oxycarbonyl) hydrazono) érythromycine (%β/%α = 0,24) et ajoute 1 mole par mole de (1,8-diazabicyclo [5-4-O] undec-7-ène. On agite 16 heures à 20°C et observe la transformation de l'isomère 10α en isomère 10β.
(%β/%α = 2,6).

### EXEMPLE 6 :

On dissout dans 2 ml de méthanol, 0,1 g d'un mélange des isomères 10α et 10β de la 11,12-didéoxy 3-de-((2,6-didéoxy 3-C-méthyl 3-O-méthyl α L-ribohexopyranosyl) oxy) 6-O-méthyl 3-oxo 12,11-(oxycarbonyl) hydrazono) érythromycine (%β/%α = 0,24) et ajoute 1 mole par mole de carbonate de potassium. On agite 16 heures à 20°C et observe la transformation de l'isomère 10α en isomère 10β.
(%β/%α = 49).

### EXEMPLE 7 :

On dissout dans 2 ml de méthanol, 0,1 g d'un mélange des isomères 10α et 10β de la 11,12-didéoxy 3-de-((2,6-didéoxy 3-C-méthyl 3-O-méthyl α L-ribohexopyranosyl) oxy) 6-O-méthyl 3-oxo 12,11-(oxycarbonyl) hydrazono) érythromycine (%β/%α = 0,24) et ajoute 1 mole par mole de phosphate de potassium. On agite 16 heures à 20°C et observe la transformation de l'isomère 10α en isomère 10β.
(%β/%α = 10,2).

### EXEMPLE 8 :

On dissout dans 2 ml de méthanol, 0,1 g d'un mélange des isomères 10α et 10β de la 11,12-didéoxy 3-de-((2,6-didéoxy 3-C-méthyl 3-O-méthyl α L-ribohexopyranosyl) oxy) 6-O-méthyl 3-oxo 12,11-(oxycarbonyl) hydrazono) érythromycine (%β/%α = 0,24) et ajoute 0,2 mole par mole de carbonate de sodium et 0,2 mole par mole de bromure de tétrabutylammonium. On agite 16 heures à 20°C et observe la transformation de l'isomère 10α en isomère 10β.
(%β/%α = 80).

### EXEMPLE 9 :

On dissout dans 0,5 ml de méthanol, 0,1 g d'un mélange des isomères 10α et 10β de la 11,12-didéoxy 3-de-((2,6-didéoxy 3-C-méthyl 3-O-méthyl α L-ribohexopyranosyl) oxy) 6-O-méthyl 3-oxo 12,11-(oxycarbonyl) hydrazono) érythromycine (%β/%α = 0,24) et ajoute 33 µl d'une solution de méthylate de sodium dans le méthanol à 5 g %ml. On agite 16 heures à 20°C et observe la transformation de l'isomère 10α en isomère 10β.

### EXEMPLE 6 :

On dissout dans 2 ml de méthanol, 0,1 g d'un mélange des isomères 10α et 10β de la 11,12-didéoxy 3-de-((2,6-didéoxy 3-C-méthyl 3-O-méthyl α L-ribohexopyranosyl) oxy) 6-O-méthyl 3-oxo 12,11-(oxycarbonyl) hydrazono) érythromycine (%β/%α = 0,24) et ajoute 1 mole par mole de carbonate de potassium. On agite 16 heures à 20°C et observe la transformation de l'isomère 10α en isomère 10β.
(%β/%α = 0,7).

## Revendications

1. Procédé d'isomérisation **caractérisé en ce que** l'on soumet un composé de formule (I_{A}) : 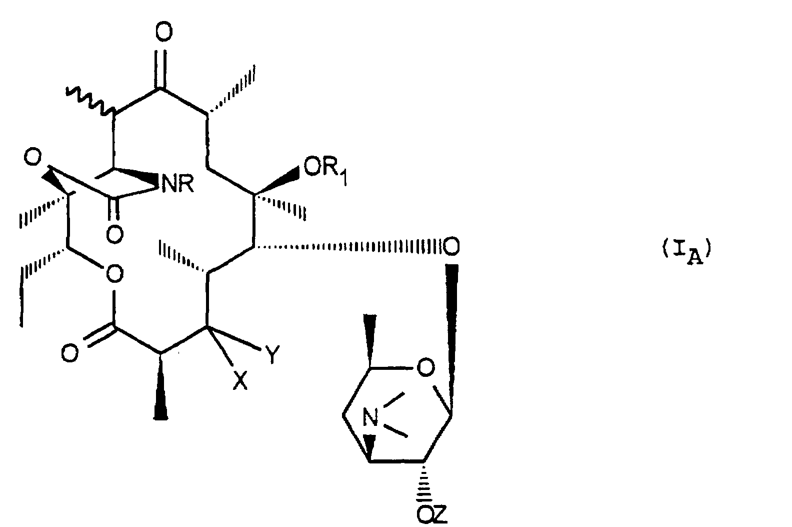 dans laquelle ou bien X et Y forment ensemble un radical 3-oxo, ou bien X représente un atome d'hydrogène et Y représente soit un radical : 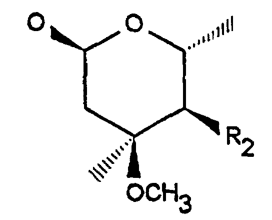 dans lequel R₂ représente un radical hydroxyle ou un radical O-acyle renfermant de 2 à 20 atomes de carbone, soit un radical O-alkyle, renfermant de 2 à 20 atomes de carbone, soit un radical NH₂,
- R₁ représente un atome d'hydrogène ou un radical méthyle,
- Z représente un hydrogène ou un radical acyle renfermant de 2 à 20 atomes de carbone,
- R représente un atome d'hydrogène, un radical NH₂ ou un radical (CH₂)ₙAr, NH(CH₂)ₙAr ou N=CH(CH₂)ₙAr dans lequel n représente un nombre entier compris entre 1 et 6, et Ar représente un radical aryle ou hétéroaryle éventuellement substitué sous forme d'isomère 10α ou de mélange d'isomères 10α et 10β à l'action d'un agent basique pour obtenir le composé de formule (I) correspondant dans lequel le radical méthyle en 10 est en position β : 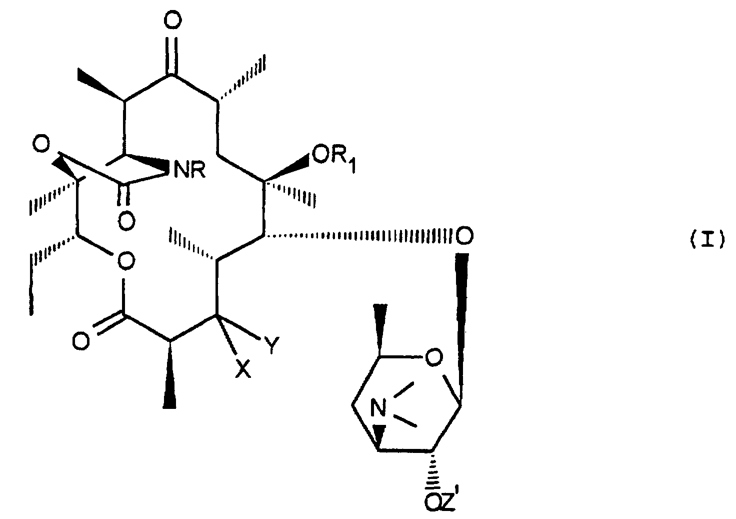 et dans laquelle R, R₁, X et Y conservent leur signification précédente et Z' représente un atome d'hydrogène ou un radical acyle renfermant de 2 à 20 atomes de carbone.

2. Procédé selon la revendication 1, **caractérisé en ce que** l'on opère en présence d'un d'agent basique, Z et Z' représentant un atome d'hydrogène.

3. Procédé selon la revendication 1 ou 2, **caractérisé en ce que** l'agent basique est la potasse.

4. Procédé selon la revendication 1 ou 2, **caractérisé en ce que** l'agent basique est l'hydroxyde ou le bromure de tétrabutylammonium ou un carbonate alcalin par exemple un carbonate de sodium ou de potassium ou la soude ou le DBU (1,8-diazabicyclo[5-4-0] undec 7-ène) ou le phosphate tripotassique ou encore le méthylate de sodium.

5. Procédé selon l'une quelconque des revendications 1 à 4, **caractérisé en ce que** l'on opère au sein d'un solvant qui peut être le tétrahydrofuranne, la 1-méthyl 2-pyrrolidinone en solution aqueuse, le chlorure de méthylène ou un alcool et obtient le composé correspondant dans lequel Z' représente un atome d'hydrogène ou un radical acyle renfermant de 2 à 20 atomes de carbone.

6. Procédé selon la revendication 5, **caractérisé en ce que** l'on opère au sein d'une solution méthanolique.

7. Procédé selon l'une quelconque des revendications 1 à 6, dans lequel R représente un radical NH₂.

8. Procédé-selon l'une quelconque des revendications 1 à 7, dans lequel R₁ représente un radical méthyle.

9. Procédé selon l'une quelconque des revendications 1 à 8, **caractérisé en ce que** X et Y forment ensemble un radical 3-oxo.

10. Procédé selon l'une quelconque des revendications 1 à 9, dans lequel Z et Z' représentent un atome d'hydrogène.

## Patentansprüche

1. Verfahren zur Isomerisierung, **dadurch gekennzeichnet, dass** man auf eine Verbindung der Formel (I_{A}): 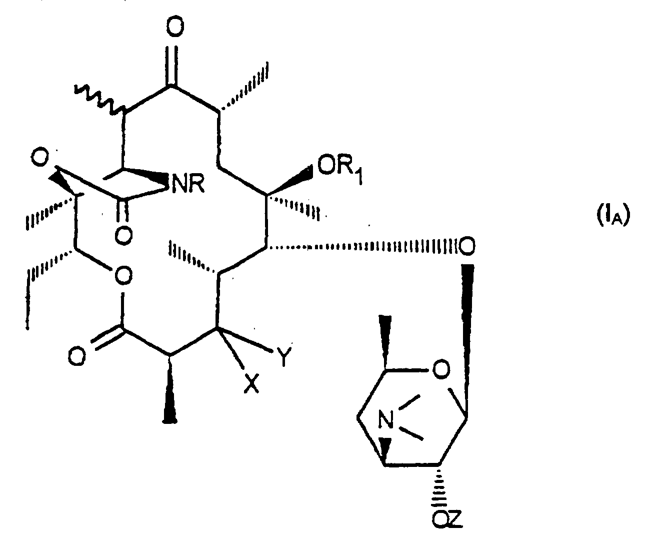 in der entweder X und Y zusammen einen 3-Oxorest bilden oder X ein Wasserstoffatom darstellt und Y entweder einen Rest: 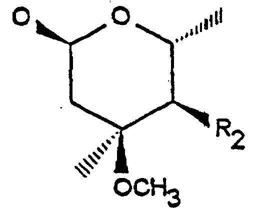 in dem R₂ einen Hydroxyrest oder einen O-Acylrest mit 2 bis 20 Kohlenstoffatomen darstellt, oder einen O-Alkylrest mit 2 bis 20 Kohlenstoffatomen oder einen NH₂-Rest darstellt,
- R₁ ein Wasserstoffatom oder einen Methylrest darstellt,
- Z ein Wasserstoffatom oder einen Acylrest mit 2 bis 20 Kohlenstoffatomen darstellt,
- R ein Wasserstoffatom, einen NH₂-Rest oder einen Rest (CH₂)ₙAr, NH(CH₂)ₙAr oder N=CH(CH₂)ₙAr darstellt, in dem n eine ganze Zahl zwischen 1 und 6 darstellt und Ar einen Aryl- oder Heteroarylrest, gegebenenfalls substituiert in Form eines 10α-Isomers oder eines Gemischs aus 10α- und 10β-Isomeren, darstellt, ein basisches Mittel einwirken lässt, um die entsprechende Verbindung der Formel (I) zu erhalten, in der sich der 10-Methylrest in β-Position befindet: 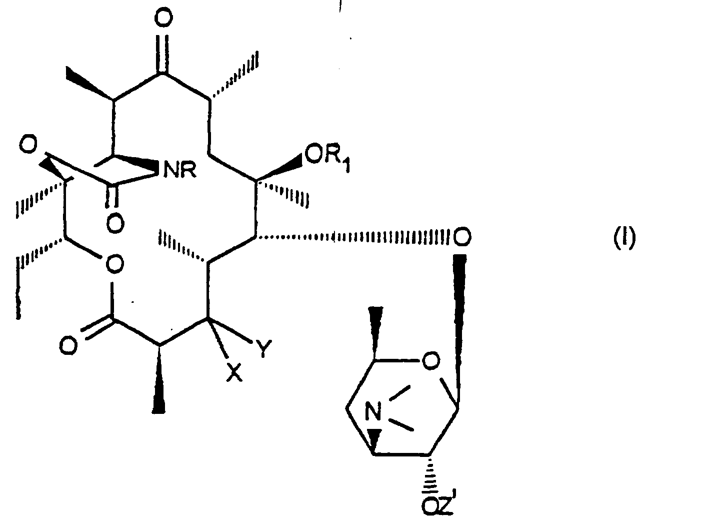 und in der R, R₁, X und Y ihre vorherige Bedeutung behalten und Z' ein Wasserstoffatom oder einen Acylrest mit 2 bis 20 Kohlenstoffatomen darstellt.

2. Verfahren gemäß Anspruch 1, **dadurch gekennzeichnet, dass** man in Gegenwart eines basischen Mittels arbeitet, wobei Z und Z' ein Wasserstoffatom darstellen.

3. Verfahren gemäß Anspruch 1 oder 2, **dadurch gekennzeichnet, dass** das basische Mittel Kaliumhydroxid ist.

4. Verfahren gemäß Anspruch 1 oder 2, **dadurch gekennzeichnet, dass** das basische Mittel Tetrabutylammonium-hydroxid oder -bromid oder ein Alkalicarbonat, beispielsweise ein Natrium- oder Kaliumcarbonat, oder Natriumhydroxid oder DBU (1,8-Diazabicyclo[5.4.0]undec-7-en) oder Trikaliumphosphat oder auch Natriummethylat ist.

5. Verfahren gemäß einem der Ansprüche 1 bis 4, **dadurch gekennzeichnet, dass** man in einem Lösungsmittel, das Tetrahydrofuran, 1-Methyl-2-pyrrolidinon in wässriger Lösung, Methylenchlorid oder ein Alkohol sein kann, arbeitet und die entsprechende Verbindung, in der Z' ein Wasserstoffatom oder einen Acylrest mit 2 bis 20 Kohlenstoffatomen darstellt, erhält.

6. Verfahren gemäß Anspruch 5, **dadurch gekennzeichnet, dass** man in einer methanolischen Lösung arbeitet.

7. Verfahren gemäß einem der Ansprüche 1 bis 6, bei dem R einen NH₂-Rest darstellt.

8. Verfahren gemäß einem der Ansprüche 1 bis 7, bei dem R₁ einen Methylrest darstellt.

9. Verfahren gemäß einem der Ansprüche 1 bis 8, **dadurch gekennzeichnet, dass** X und Y zusammen einen 3-Oxorest bilden.

10. Verfahren gemäß einem der Ansprüche 1 bis 9, bei dem Z und Z' ein Wasserstoffatom darstellen.

## Claims

1. Process of isomerization **characterized in that** a compound of formula (I_{A}): 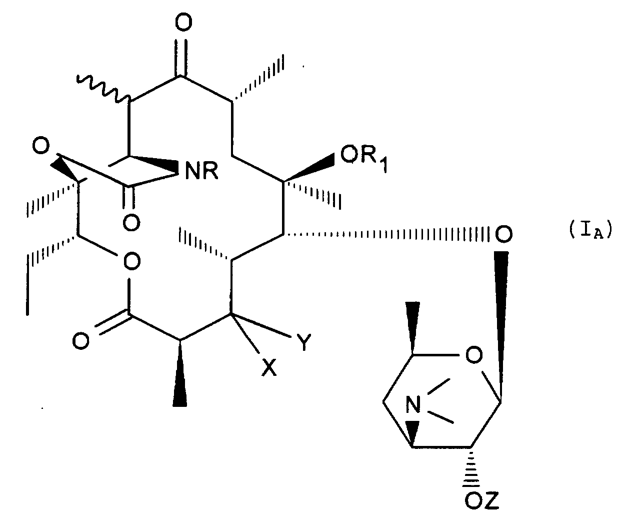 in which either X and Y together form a 3-oxo radical, or X represents a hydrogen atom and Y represents either a: 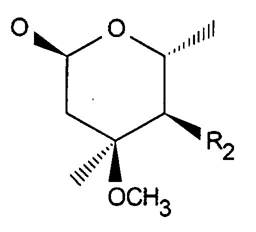 radical in which R₂ represents a hydroxyl radical or an O-acyl radical containing from 2 to 20 carbon atoms, or an O-alkyl radical, containing from 2 to 20 carbon atoms, or an NH₂ radical,
- R₁ represents a hydrogen atom or a methyl radical,
- Z represents a hydrogen or an acyl radical containing from 2 to 20 carbon atoms,
- R represents a hydrogen atom, an NH₂ radical or a (CH₂)ₙAr, NH(CH₂)ₙAr or N=CH(CH₂)ₙAr radical in which n represents an integer comprised between 1 and 6, and Ar represents an aryl or heteroaryl radical optionally substituted in the form of a 10α isomer or of mixture of 10α and 10β isomers, is subjected to to the action of a basic agent in order to obtain the corresponding compound of formula (I) in which the methyl radical in position 10 is in β position: 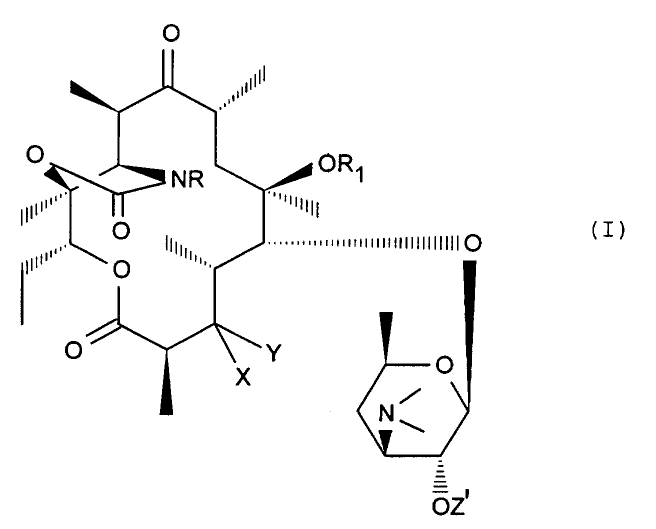 and in which R, R₁, X and Y retain their previous meaning and Z' represents a hydrogen atom or an acyl radical containing from 2 to 20 carbon atoms.

2. Process according to claim 1, **characterized in that** it is carried out in the presence of a basic agent, Z and Z' representing a hydrogen atom.

3. Process according to claim or 2, **characterized in that** the basic agent is potash.

4. Process according to claim or 2, **characterized in that** the basic agent is tetrabutylammonium hydroxide or bromide or a alkaline carbonate for example sodium or potassium carbonate or soda or DBU (1,8-diazabicyclo[5-4-0] undec 7-ene) or tripotassium phosphate or also sodium methylate.

5. Process according to any one of claims 1 to 4, **characterized in that** it is carried out in a solvent which can be the tetrahydrofuran, 1-methyl 2-pyrrolidinone in aqueous solution, methylene chloride or an alcohol and the corresponding compound is obtained in which Z' represents a hydrogen atom or an acyl radical containing from 2 to 20 carbon atoms.

6. Process according to claim 5 **characterized in that** it is carried out in a methanolic solution.

7. Process according to any one of claims 1 to 6, in which R represents an NH₂ radical.

8. Process according to any one of claims 1 to 7, in which R₁ represents a methyl radical.

9. Process according to any one of claims 1 to 8, **characterized in that** X and Y together form a 3-oxo radical.

10. Process according to any one of claims 1 to 9, in which Z and Z' represent a hydrogen atom.
